# EUROPÄISCHE PATENTSCHRIFT

(11) **EP 1 568 337 B1**
(45) Veröffentlichungstag und Bekanntmachung des Hinweises auf die Patenterteilung: **15.11.2006**
(21) Anmeldenummer: 05003742.3
(22) Anmeldetag: 22.02.2005
(51) Int. Cl.: A61F 5/01

(54) **Orthese zur Korrektur der Stellung eines Körpergelenks**
Orthosis for the correction of the position of an anatomical joint
Orthèse pour la correction de la position d'une articulation anatomique

(30) Priorität: 24.02.2004 DE 102004008909
(43) Veröffentlichungstag der Anmeldung: 31.08.2005
(73) Patentinhaber: Bauerfeind AG, 07937 Zeulenroda (DE)
(72) Erfinder: Reinhardt, Holger, 47906 Kempten (DE)
(74) Vertreter: Bardehle, Heinz

(56) Entgegenhaltungen:
- US-A- 3 671 978
- US-A- 5 086 760
- US-A- 6 027 466
- US-B1- 6 254 559

## Beschreibung

Die Erfindung bezieht sich auf eine der Korrektur der Stellung eines Körpergelenks dienende Orthese, die sich über die durch das Körpergelenk verbundenen beiden Glieder erstreckt und mit zwei die Orthese bildenden Schenkeln versehen ist, die an den Gliedern durch jeweils ein Befestigungsmittel gehalten sind und durch ein dem Körpergelenk benachbarten Drehgelenk miteinander verbunden sind.

Eine derartige Orthese für das Kniegelenk ist in der europäischen Patentschrift EP 0 879 032 B1 beschrieben und dargestellt. Die beiden Schenkel der Orthese sind hier durch eine zweiteilige Schiene gebildet, die ein gegenüber dem Kniegelenk in Anpassung an dessen Bewegungsablauf zurückversetztes Gelenk mit einer Achse aufweist, die aufgrund einer exzentrischen Lagerung in einem Drehlager die beiden Schienenteile drehbeweglich verbindet und durch die Verdrehung des Drehlagers die Achse des Gelenks verschiebt, um damit dieser eine Lage zu geben, die weitgehend dem Bewegungsablauf des Kniegelenks angepasst ist.

Es ist weiterhin eine Orthese zur Korrektur der Stellung der Großzehe aus der deutschen Gebrauchsmusterschrift DE 203 12 159 U1 bekannt. Die Orthese dient dazu, mit einer Gelenkbiegeschiene, die sich vom Mittelfuß zur Großzehe an der Innenseite des Fußes erstreckt, der Großzehe eine von der Gelenkbiegeschiene bestimmte Ausrichtung zu geben, um damit eine Fehlstellung der Großzehe zu beheben, die in einer Schrägstellung der Großzehe zur Längsrichtung des Fußes besteht. Als besonders günstig wird hierzu auf Seite 9 letzter Absatz der Druckschrift eine kohlefaser-verstärkte Platte für die Gelenkbiegeschiene herausgestellt, die unter Hitzeeinwirkung formbar ist und nach dem Abkühlen eine große Feder kraft aufweist. Von dem Material der Gelenkbiegeschiene wird dabei eine die Stellung der Großzehe korrigierende Kraft ausgeübt, wobei die Beweglichkeit der Großzehe in der natürlichen Flexions-Extensionsrichtung, also der beim Fußabrollen erforderlichen Beugung der Großzehe, dadurch nicht eingeschränkt wird, dass die bekannte Orthese ein sich um eine Achse drehbares Gelenk aufweist, das diese Beugung bzw. Streckung ermöglicht. Die durch eine thermische Veränderung der Ausrichtung der Gelenkbiegeschiene ermöglichte individuelle Anpassung der Gelenkbiegeschiene erfordert einen erheblichen Aufwand, da hierzu wie oben gesagt, eine Erhitzung des Materials der Gelenkbiegeschiene erfolgen muss, die vom Fachmann wegen der dabei auftretenden notwendigen Temperaturen nicht am Patienten selbst vorgenommen werden kann. Es muss also gewissermaßen versuchsweise eine solche Anpassung erfolgen, ggf. durch mehrfaches Erhitzen der Gelenkbiegeschiene und jeweiliges Nachbiegen. Dabei ist jedes Mal das An- und Ablegen der Gelenkbiegeschiene am Patienten erforderlich. Für eine im Laufe der Zeit ggf. erforderlichen Nachstellung der Gelenkbiegeschiene ist wiederum der Fachmann aufzusuchen, der über die notwendigen Mittel verfügt, um die Gelenkbiegeschiene entsprechend nachzubiegen. Wegen der Unmöglichkeit, diese Anpassung am Fuß des Patienten vorzunehmen, kann diese praktisch nur nach Augenmaß erfolgen, was vor allem ein späteres Nachjustieren der Gelenkbiegeschiene erschwert, da hierbei nicht auf vorangegangene Ausgangswerte Rücksicht genommen werden kann, da diese normalerweise überhaupt nicht bekannt sind.

Der Erfindung liegt daher die Aufgabe zugrunde, eine Orthese für die Korrektur der Stellung eines Körpergelenks zu schaffen, mit der bei Aufrechterhaltung der normalen Beugebeweglichkeit des Gelenks wahlweise eine gewünschte, als notwendig erkannte Einstellung der Beugebewegungsebene vorgenommen werden kann, die dann später auch ohne weiteres am Patienten nachjustierbar ist.

Die US-Patentschrift US-B-6,254,559 offenbart eine Orthese mit zwei Schenkeln und mit einem Drehgelenk, das zwei Keilscheiben umfaßt, wobei durch relatives Verdrehen der Keilscheiben zueinander ein Schenkel bezüglich eines anderen Schenkels geschwenkt werden kann.

In der erfindungsgemäßen Orthese, wie sie eingangs beschrieben ist, geschieht dies durch eine derartige Gestaltung, dass das Drehgelenk für eine bei Normalbewegung erforderliche Beugung durch einen von dem einen Schenkel umfassten Achsring gebildet ist, der mit einem zum Achsring exzentrischen Ringlager zu einem zentralen Einstellring fest verbunden ist, welches Ringlager ein von dem anderen Schenkel umfasstes Drehlager für diesen Schenkel bildet und dessen Drehebene zur Drehebene des Achsringes durch Verdrehen des Einstellrings um einen solchen Winkel verschwenkbar ist, dass der von dem Achsring getragene Schenkel und mit ihm das von ihm gehaltene Glied je nach Verdrehwinkel des Einstellrings gegen den mit dem Ringlager verbundenen Schenkel eine Schwenklage dieses Gliedes gegenüber der gestreckten Normallage einnimmt.

Durch diese Gestaltung wird konstruktiv in die eine Beugung ermöglichende Bewegung des einen Schenkels eine Verschwenkung dieses Schenkels einbezogen, die sich stufenlos über den Bereich einstellen lässt, der für die Behandlung der Fehlstellung eines Gliedes des Körpergelenks erforderlich ist. Für diese Einstellung ist lediglich die Verdrehung des den betreffenden Schenkel tragende Ringlagers erforderlich, das bei seiner Verdrehung aufgrund seiner Exzentrizität zum Ringlager seine Drehebene gegenüber der Drehebene des Achsringes (Beugebewegungsebene) verändert und dabei einen jeweils gewünschten Winkel einnimmt, der sich dann in einer entsprechenden Schwenklage des auf das betreffende Glied einwirkenden Schenkels auswirkt, dessen Beugebewegungsebene damit entsprechend verschwenkt ist. Damit kommt der konstruktiven Kombination dieser beiden erforderlichen Verdrehungen in dem zentralen Einstellring, umfassend das mit dem Achsring fest verbundenen Ringlager, die entscheidende Bedeutung zu, da der Einstellring über den in diesem Zusammenhang erforderlichen Winkelbereich für die Schwenklage des betreffenden Gliedes einem Kardangelenk ähnlich wirkt, allerdings ohne den Aufwand einer zweiten Achse, wobei wegen der festen Verbindung zwischen Achsring und Ringlager eine in seitlicher Richtung besonders gedrungene Konstruktion geschaffen ist, die das Tragen der Orthese beschwerdelos ermöglicht.

Die erfindungsgemäße Orthese kann im Zusammenhang mit allen Körpergelenken verwendet werden, die eine Beugebewegung auszuführen haben, bei denen die Beugebewegungsebene ggf. hinsichtlich ihres Winkels zur normalen Beugebewegungsebene verstellt werden soll. Dies ist z.B. zur Korrektur der Stellung der Großzehe oder der Korrektur der Stellung des Kniegelenks erforderlich, aber natürlich an anderen Körpergelenken ebenfalls möglich.

Eine Orthese zur Korrektur der Stellung der Großzehe mit zwei Schenkeln, die sich vom Mittelfuß zur Großzehe an der Innenseite des Fußes erstrecken und am Mittelfuß und an der Großzehe durch jeweils eine Manschette gehalten sind, gestaltet man zweckmäßig derart, dass das Drehgelenk für eine beim Fußabrollen erforderliche Beugung durch den von dem einen Schenkel umfassten Achsring gebildet ist und das von dem anderen Schenkel umfasste Ringlager je nach Verdrehwinkel des Einstellrings eine Schwenklage des ersteren Schenkels gegenüber der gestreckten Normallage bestimmt.

Durch die Verdrehung des Einstellringes lässt sich dann in dem für die Korrektur der Stellung der Großzehe erforderlichen Bereich eine Einstellung der Beugebewegungsebene herbeiführen und damit eine Fehlstellung der Großzehe entsprechend korrigieren. Besonders vorteilhaft ist dabei, dass sich diese Einstellung bei angelegter Orthese durchführen lässt.

Ein weiteres Beispiel für die Anwendung der erfindungsgemäßen Orthese ist die Korrektur der Stellung des Kniegelenks mittels zweier Schenkel der Orthese, die sich vom Oberschenkel über das Kniegelenk zum Unterschenkel erstrecken, am Oberschenkel und Unterschenkel durch jeweils eine Manschette gehalten sind und durch ein dem Kniegelenk benachbartes Drehgelenk miteinander verbunden sind. Zweckmäßig wird diese Orthese derart gestaltet, dass das Drehgelenk für eine bei der Kniegelenkbewegung erforderliche Beugung durch den von dem einen Schenkel umfassten Achsring gebildet ist und das von dem anderen Schenkel umfasste Ringlager je nach Verdrehwinkel des Einstellrings eine Schwenklage des ersteren Schenkels gegenüber der gestreckten Normallage bestimmt.

Durch die jeweilige Einstellung des Einstellringes lässt sich eine sowohl dem X-Bein als auch dem O-Bein entgegenwirkende Einstellung der Orthese ermöglichen, und zwar auch in dem Falle bei angelegter Orthese.

Um dem Patienten bzw. dem behandelnden Arzt deutlich sichtbar zu machen, welche Schwenklage der die Großzehe beeinflussende Schenkel einnimmt, lässt sich der den Achsring umfassende Schenkel mit einer Ringskala versehen, die den Verdrehwinkel des Ringlagers und damit die Schwenklage des an der Großzehe anliegenden Schenkels anzeigt. Die Verdrehung des Ringlagers, bei der der Achsring in dem ihn umfassenden Schenkel sich gleitend dreht, lässt sich mit irgendeinem Werkzeug leicht durchführen, das irgendwie in das Ringlager eingreift. Hierzu wird weiter unten ein Beispiel angegeben.

Um eine gesicherte Verbindung zwischen den beiden Schenkeln einerseits und dem Einstellring herbeizuführen, kann man vorteilhaft einen in den Achsring eingedrückten Spreizring verwenden, der durch die Verspreizung im Sinne des Achsrings oder des Ringlagers dafür sorgt, dass die Verbindung der genannten Teile sich nicht lösen kann, solange der Spreizring eingedrückt ist. Dieser Spreizring kann auch dazu benutzt werden, mit seinem Rand in Ausnehmungen im Ringlager einzugreifen, wie dies in bekannter Weise bei elektronischen Geräten mittels einer Münze geschieht. Mit dem Spreizring wird der jeweils gewünschte Verdrehwinkel des Ringlagers eingestellt, womit dann die notwendige Schwenklage des an der Großzehe anliegenden Schenkels eingestellt ist. Danach wird dann der Spreizring in den Achsring eingedrückt, womit die ganze Verbindung gesichert ist.

Um auch in dem Fall die Orthese mit ihrer Ausrichtwirkung auf die Großzehe wirksam verwenden zu können, in dem aus medizinischen Gründen ein Fußabrollen nicht geschehen darf, z.B. nach einer vorangegangenen Operation, also die Großzehe stets in gerader Ausrichtung festgehalten werden soll, ohne eine Beugung durchführen zu können, versieht man den den Achsring umfassenden Schenkel zweckmäßig mit einer wahlweise anzubringenden Verdrehsicherung, die den Schenkel und den Achsring so miteinander verriegeln, dass der Schenkel sich um den Achsring nicht mehr drehen kann. Dies kann beispielsweise durch irgendeine wahlweise einrastbare Verzahnung geschehen. Hierdurch wird erreicht, dass bei Aufrechterhaltung der gewünschten Schwenklage des an der Großzehe anliegenden Schenkels dieser nicht um seinen Achsring gedreht werden kann, also die Großzehe in ihrer eingestellten Lage festgehalten wird, was dann so lange geschieht, wie die Verdrehsicherung angebracht ist.

Um das Tragen der Orthese so zu gestalten, dass dabei kein unangenehm wirkender Druck ausgeübt wird, kann man die Schenkel auf ihrer dem Körperglied zugewandten Seite vorteilhaft mit einer entsprechenden Polsterung versehen.

Zweckmäßig werden der Achsring und das Ringlager einstückig ausgebildet. Dabei kann es sich insbesondere um ein Kunststoffspritzteil handeln, das sich einfach herstellen lässt und das auch die notwendige Festigkeit bzw. Elastizität für Sicherungsmaßnahmen besitzt.

### In den Figuren ist ein Ausführungsbeispiel der Erfindung dargestellt. Es zeigen:

- Fig. 1: Die an einem rechten Fuß angebrachte Orthese;
- Fig. 2: die gleiche Orthese bei abgebeugten Zehen;
- Fig. 3: den für die Stellung der Großzehe verantwortlichen Einstellring;
- Fig. 4 a, b, c: das Gelenk der Orthese im Schnitt in einer Einstellung des Einstellrings zur Korrektur der Großzehe eines rechten Fußes bei nach links ausgelenkter Großzehe;
- Fig. 5 a, b, c: das gleiche Gelenk im Querschnitt mit derart verdrehtem Einstellring, dass die Stellung der Großzehe eines rechten Fußes normal gehalten wird;
- Fig. 6 a, b, c: das gleiche Gelenk mit eingestelltem Einstellring für einen rechten Fuß mit nach rechts ausgelenkter Großzehe;
- Fig. 7: eine Explosionszeichnung der wesentlichen Bestandteile des Gelenks, wie sie in den Figuren 4 bis 6 dargestellt sind;
- Fig. 8: die an einem Bein angebrachte Orthese in perspektivischer Sicht;

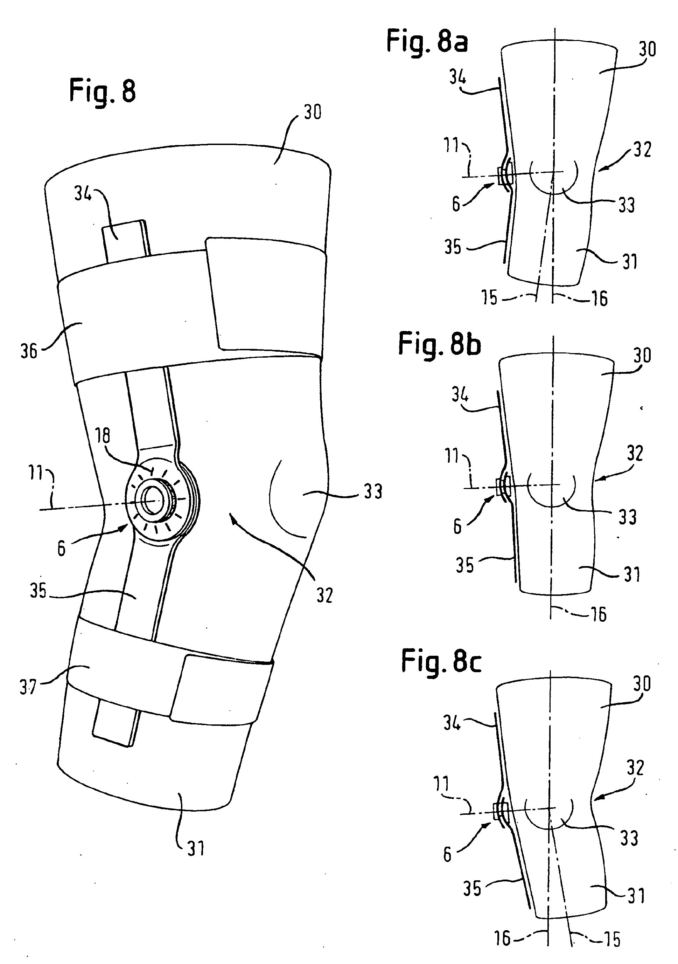

- Fig. 8 a, b, c: die gleiche Orthese am Bein angebracht in einer Sicht auf das Kniegelenk, wobei aus Gründen der Vereinfachung der Darstellung die Manschetten für die Halterung der Orthese weggelassen sind.

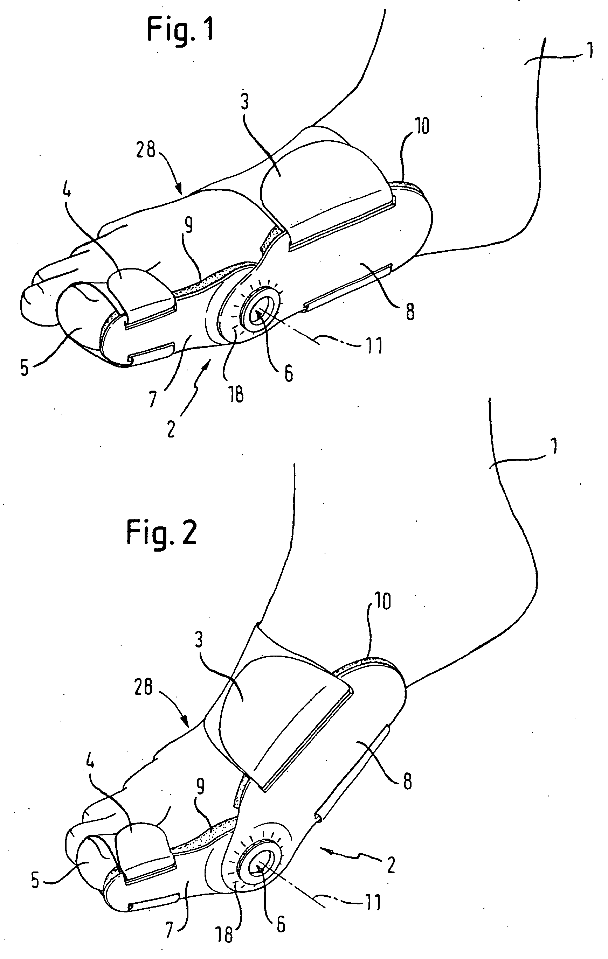

In der Figur 1 ist die an einem rechten Fuß 1 angebrachte Orthese 2 in einer Position dargestellt, in der die Orthese 2 durch die festlegbare Bandage 3 am Mittelfuß 28 und die ebenfalls festlegbare Bandage 4 an der Großzehe 5 in Normalstellung des Fußes angelegt ist. Die Orthese 2 ist mit dem etwa in ihrer Mitte befindlichen Gelenk 6 versehen, das die Orthese 2 in den Zehenschenkel 7 und den Mittelfußschenkel 8 aufteilt. Sowohl der Zehenschenkel 7 als auch der Mittelfußschenkel 8 sind an ihrer dem Fuß zugewandten Seite mit einer Polsterung 9 und 10 versehen, die für ein beschwerdeloses Tragen der Orthese 2 sorgen. Bei den beiden Bandagen 3 und 4 handelt es sich um bekannte Gestaltungen. Dabei sorgt die Bandage 3 für die Halterung des Mittelfußschenkels 8 und die Bandage 4 für die Halterung des Zehenschenkels 7.

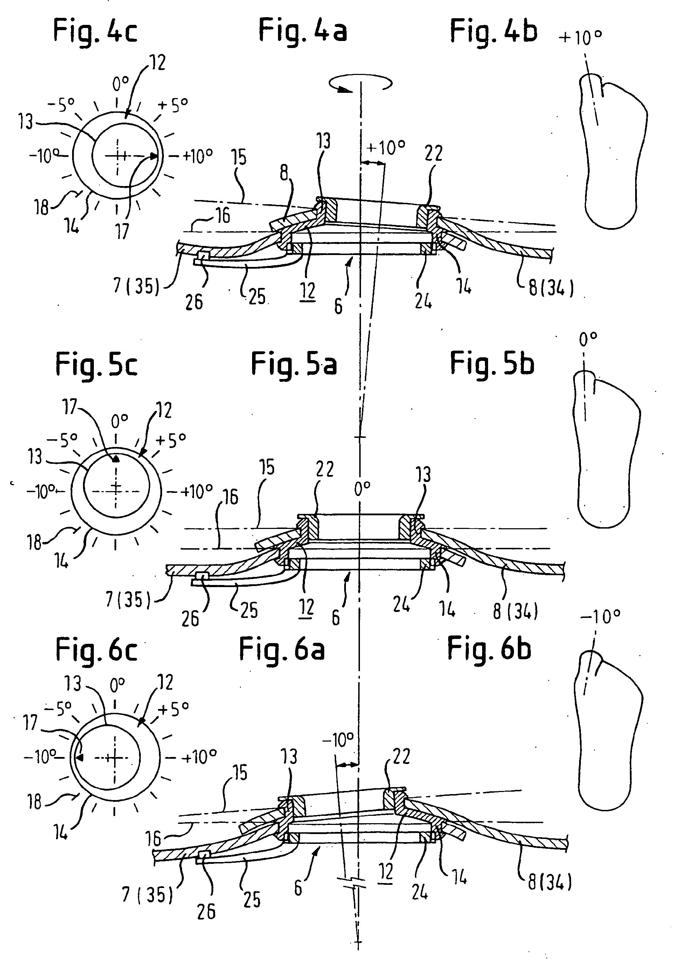

Das Gelenk 6 ermöglicht es der Orthese 2, beim Abrollen des Fußes 1, wie in Figur 2 dargestellt, die Zehen und damit die Großzehe 5 gegenüber dem Mittelfuß 28 zu beugen. Das Gelenk 6 besitzt hierzu eine drehbare Gestaltung, auf die im Zusammenhang mit den Figuren 4 bis 6 näher eingegangen wird. Die Wirkung als Drehlager wird in den Figuren 1 und 2 durch die in dem Gelenk 6 eingezeichnete strichpunktierte Linie 11 dargestellt.

Die Gestaltung des Gelenks 6 gemäß den Figuren 1 und 2 ist in den Figuren 4 bis 6 näher dargestellt, wobei verschiedene Einstellungen des Gelenks 6 gezeichnet sind. Das Gelenk 6 enthält einen Einstellring 12, in dem das Ringlager 13 für den Mittelfußschenkel 8 mit dem Achsring 14 für den Zehenschenkel 7 zusammengefasst sind. Der Einstellring 12 ist hierzu einstückig mit dem Achsring 14 und dem Ringlager 13 verbunden.

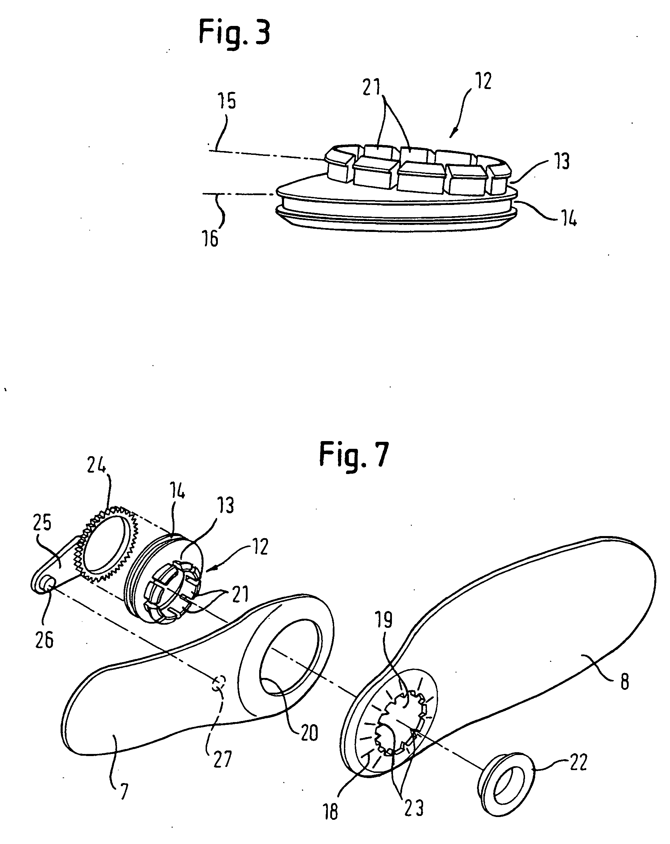

Zur Veranschaulichung der Gestaltung und Funktion des Einstellringes 12 sei auf die Figur 3 verwiesen, die den Einstellring 12 hinsichtlich seiner besonderen Gestaltung deutlich zeigt, vor allem im Hinblick auf die Schräglage von Drehebene 15 des Ringslagers 13 zur Drehebene 16 des Achsrings 14. Bei Verdrehung des Einstellrings 12 wandert der Teil mit dem Ringlager 13 gemäß seiner Exzentrizität gewissermaßen um das Zentrum des Einstellrings 12 herum, wobei er aufgrund der sich dabei ergebenden Änderung der Lage der Drehebene 15 von der einen extremen Winkellage (siehe Figur 4 a) zu der anderen extremen Winkellage (siehe Figur 6 a) gelangt.

Die Lagerung des Mittelfußschenkels 8 und des Zehenschenkels 7 in dem betreffenden Teil des Einstellringes 12, also dem Achsring 14 und dem Ringlager 13, erfolgt, wie die Figuren 4 a, 5 a und 6 a deutlich zeigen, durch das Übergreifen eines Randes des Achsrings 14 bzw. des Ringlagers 13 über die entsprechende kreisförmige Ausnehmung im Mittelfußschenkel 8 bzw. Zehenschenkel 7 (siehe auch Fig. 7), so dass sich sowohl der Zehenschenkel 7 als auch der Mittelfußschenkel 8 gegenüber dem Einstellring 12 verdrehen lassen können. Die Zusammenfügung von Zehenschenkel 7 und Mittelfußschenkel 8 in dem Einstellring 12 erfolgt dabei dadurch, dass der jeweilige Schenkel auf die betreffende Lagerung, also Achsring 14 bzw. Ringlager 13, aufgerastet werden, wobei aufgrund einer entsprechenden Elastizität des Einstellringes 12 der betreffende übergreifende Rand zurückweicht, der hierzu mit einer außen ansetzenden Schräge versehen ist, womit also die beiden Schenkel 7 und 8 mit dem Einstellring 12 so miteinander verbunden werden können, dass der Einstellring 12 die beiden Schenkel 7 und 8 fest, aber gegenüber dem Einstellring 12 verdrehbar, hält.

Der Einstellring 12 lässt sich gegenüber dem Mittelfußschenkel 8 durch Verdrehung des Ringlagers 13 wahlweise einstellen, wobei sich aufgrund der Exzentrizität von Ringlager 13 zum Achsring 14 und einer Schrägstellung der Drehebene 15 des Ringlagers 13 zur Drehebene 16 des Achsrings 14 ein jeweiliger Verschwenkwinkel des Zehenschenkels 7 ergibt, wie in der Folge der Figuren 4 a, 5 a und 6a dargestellt ist, und zwar von +10° zu einem Winkel von -10° für die Halterung des Zehenschenkels 7, der dabei von einer in Figur 4 b dargestellten vom Fuß wegweisenden Spreizstellung über seine Normalstellung gemäß Figur 5 b in eine zum Fuß nach innen gerichtete Spreizstellung gemäß Figur 6 b verlagert wird. Durch diese sich dabei ergebende Verschiebung der Drehebene 15 ergibt sich die vorstehend behandelte Verstellung des Zehenschenkels 7, der damit aufgrund seiner festen Anlage an der Großzehe 5 diese in eine entsprechende Stellung verschwenkt.

Damit ergibt sich eine Lagerung für den Zehenschenkel 7, die durch Verdrehung des Einstellringes 12 jeweils die im Zusammenhang mit den Figuren 4 b, 5 b und 6 b dargestellte Lage der Großzehe bestimmt, wobei die Verdrehung innerhalb des Gelenkes bei der Beugung der Zehen (siehe Figur 2) sich im Bereich der Verbindung von Achsring 14 mit dem Zehenschenkel 7 abspielt. Bei dieser Verdrehung aufgrund der genannten Beugung bleibt also die Halterung des Mittelfußschenkels 8 in dem Ringlager 13 unverändert, so dass unabhängig von dem Grad der jeweiligen Beugung die Einstellung des Zehenschenkels 7 gemäß der vorher erfolgten Einstellung von Einstellring 12 zum Mittelfußschenkel 8 erhalten bleibt.

In den Figuren 4 c, 5 c und 6 c sind die jeweiligen Einstellungen des Einstellringes 12 in Bezug auf den Mittelfußschenkel 8 schematisch dargestellt. Dabei symbolisiert der innere Kreis das Ringlager 13 und der äußere Kreis den Achsring 14. Auf dem Mittelfußschenkel 8 ist um den Einstellring 12 herum eine Skala 18 mit Winkelgraden angegeben, wie sie auch in den Figuren 1 und 2 prinzipiell dargestellt ist. Gegenüber dieser Skala 18 ist am Ringlager 13 eine Markierung 17 angebracht, die bei Hinweis auf einen entsprechenden Wert der Skala 18 angibt, welcher Verschwenkungsgrad der Zehenschenkel 7 gegenüber der Normallage (0°) eingenommen hat. Der Zehenschenkel 7 wird dabei entsprechend verschwenkt (siehe die betreffenden Gradangaben in den Figuren 4 b, 5 b und 6 b) und behält dann diese Einstellung auch bei, unabhängig davon, ob sich aufgrund einer Beugung der Zehen eine Verdrehung des Einstellringes 12 gegenüber dem Zehenschenkel 7 ergibt, die sich im Bereich des Achsrings 14 abspielt.

In der Figur 7 ist in einer Explosionszeichnung der Aufbau des Gelenks 6 dargestellt, wobei nebeneinander der Mittelfußschenkel 8 und der Zehenschenkel 7 gezeichnet ist, die mit ihren fluchtenden Ausnehmungen 19 und 20 zusammengehalten werden. Dies geschieht durch den Einstellring 12, der aus dem Ringlager 13 und dem Achsring 14 besteht. Beim Zusammenfügen von Einstellring 12, Zehenschenkel 7 und Mittelfußschenkel 8 durchsetzt das Ringlager 13 die Ausnehmungen 20 und 19, bis sich schließlich aufgrund der Elastizität der einzelnen Rippen 21 des Ringlagers 13 dieses hinter dem betreffenden Rand der Ausnehmung 19 im Mittelfußschenkel 8 verrastet, womit die genannten drei Teile miteinander verbunden sind. Um diese Verbindung zu sichern, ist der Spreizring 22 vorgesehen, der in die Bohrung im Ringlager 13 passt und dabei dessen einzelne Rippen nach außen drückt. Der Spreizring 22 sorgt darüber hinaus dafür, dass eine eingestellte Winkellage des Einstellringes 12 zum Mittelfußschenkel 8 gesichert bleibt. Dies geschieht dadurch, dass in der Ausnehmung 19 des Mittelfußschenkels 8 Vosprünge 23 vorgesehen sind, die in die Lücken zwischen den einzelnen Rippen 21 im Ringlager 13 eingreifen. Dieser Eingriff wird durch Abnahme des Spreizrings 22 gelöst, so dass sich bei abgenommenem Spreizring 22 die im Zusammenhang mit den Figuren 4, 5 und 6 dargestellte Einstellung des Einstellringes 12 ermöglichen lässt. Nach vorgenommener Einstellung wird dann der Spreizring 22 eingesetzt, womit der eingestellte Verschwenkungsgrad für den Zehenschenkel 7 erhalten bleibt.

In der Figur 7 ist noch eine weitere Sicherung dargestellt, und zwar in Form der Verdrehsicherung 24, die in die Ausnehmung im Achsring 14 eindrückbar ist und sich dort mit ihrer Verzahnung in der Ausnehmung festkrallt. Durch diese Verdrehsicherung 24 wird erreicht, dass, falls dies aus therapeutischen Gründen erforderlich ist, eine Beugung des Zehenschenkels 7 verhindert wird, was ggf. für einen kurzen Zeitraum nach einer Operation erforderlich ist. Hierzu wird dann die Verdrehsicherung 24 mit ihrem Ausleger 25 und dem Feststellknopf 26 in ein entsprechendes Loch 27 im Zehenschenkel 7 eingesetzt, womit für die Zeit des Einsatzes der Verdrehsicherung 24 auch eine Feststellung des Zehenschenkels 7 herbeigeführt wird (siehe auch Fig. 4a, 5a, 6a).

Die erfindungsgemäße Orthese, die in den Figuren unter Bezugnahme auf einen rechten Fuß dargestellt ist, lässt sich natürlich auch auf einen linken Fuß übertragen, wobei gewissermaßen spiegelbildlich die Orthese an einem linken Fuß anzubringen ist, ohne dass sich am inneren Aufbau der Orthese etwas ändert. Die aus den Figuren 4 c, 5 c und 6 c ersichtliche Verdrehung des Einstellringes 12 verläuft über den in den Figuren gezeichneten oberen Teil der Skala 18 und war damit auf einen rechten Fuß bezogen. Wenn die Orthese an einem linken Fuß angebracht wird, ergibt sich für die Benutzung der Skala deren unterer Bereich, der gewissermaßen spiegelbildlich zu dem oberen Bereich liegt. Es ist daher mit einer in den Mittelfußschenkel eingravierten Rundumskala möglich, diese sowohl im Zusammenhang mit einem rechten Fuß als einem linken Fuß zu verwenden. Genauso ist es möglich, das im Ausführungsbeispiel auf der Außenseite dargestellte Ringlager und den auf der Innenseite dargestellten Achsring auszutauschen, so dass also der Achsring außen angeordnet sein würde. In beiden Fällen verbleibt es bei einer Zuordnung von dem einen Schenkel zum Achsring und dem anderen Schenkel zum Ringlager bzw. umgekehrt, ohne dass sich dabei an der Funktion des Gelenks etwas ändert.

In den Figuren 8 und 8a bis c ist ein weiteres Ausführungsbeispiel für die Anwendung der erfindungsgemäßen Orthese dargestellt. Es handelt sich dabei um eine Kniegelenkorthese, die am Oberschenkel 30 und Unterschenkel 31 des Beines angelegt ist, das das Kniegelenk 32 enthält, wozu die Kniescheibe schematisch durch das Bezugszeichen 33 angedeutet ist. Die Kniegelenkorthese besitzt die beiden Schenkel 34 und 35, die jeweils von den als Manschetten 36 und 37 ausgebildeten Befestigungsmitteln am Oberschenkel 30 und am Unterschenkel 31 angeschnallt sind. Bei den Manschetten handelt es sich um bekannte Gestaltungen. Die beiden Schenkel 34 und 35 sind durch das Drehgelenk 6 miteinander verbunden, das hinsichtlich seiner inneren Gestaltung vollständig dem Drehlager 6 gemäß den Figuren 1 bis 7 entspricht. Insbesondere wird hierzu auf die Figuren 4 a, b, c, 5 a, b c und 6 a, b, c verwiesen, deren Stellungen in prinzipieller Weise in den Figuren 8 a, b und c ebenfalls auftreten. Bezüglich der Beugebewegung des Kniegelenks 32 und der jeweiligen seitlichen Verschwenkung des Kniegelenks 32 sei auf die Darstellungen in den Figuren 4 a, 4 c, 5 a, 5 c, 6 a, 6 c verwiesen.

In den Figuren 8 a, 8 b und 8 c sind die in den Figuren 4 a, 5 a und 6 a eingezeichneten Drehebenen 15 und 16 ebenfalls wiedergegeben, allerdings seitlich versetzt in Richtung zur Mitte des Kniegelenks 32. Die in den Figuren 8 a und 8 c dargestellte Verschiebung der Drehebene 16 ergibt sich aufgrund einer entsprechenden Verdrehung des in dem Drehgelenk 6 verdrehten Einstellrings, womit die Lage des Schenkels 35 gegenüber dem Schenkel 34 entsprechend verstellt wird, wie eine Gegenüberstellung von Figur 8 a und 8 c deutlich zeigt. Dabei fällt die Drehebene 15 in Normalstellung gemäß Figur 8 b mit der Drehebene 16 zusammen, die für die normale Beugebewegung des Kniegelenks 32 verantwortlich ist.

Bezüglich der Verwendung am rechten oder linken Kniegelenk gilt einerseits das oben im Zusammenhang mit dem rechten oder linken Fuß Gesagte. Jedoch ist es natürlich ohne weiteres möglich, die erfindungsgemäße Orthese am Kniegelenk innen oder außen anzubringen, was durch die niedrige Bauhöhe der Orthese mit ihrem Drehgelenk 6 ermöglicht wird.

## Patentansprüche

1. Der Korrektur der Stellung eines Körpergelenks dienende Orthese (2), die sich über die durch das Körpergelenk verbundenen beiden Glieder (5, 28; 30, 31) erstreckt und mit zwei die Orthese (2) bildenden Schenkeln (7, 8; 34, 35) versehen ist, die an den Gliedern (5, 28; 30, 31) durch jeweils ein Befestigungsmittel (3, 4; 36, 37) gehalten sind und durch ein dem Körpergelenk benachbarten Drehgelenk (6) miteinander verbunden sind, **dadurch gekennzeichnet, dass** das Drehgelenk (6) für eine bei Normalbewegung erforderliche Beugung durch einen von dem einen Schenkel (7, 35) umfassten Achsring (14) gebildet ist, der mit einem zum Achsring (14) exzentrischen Ringlager (13) zu einem zentralen Einstellring (12) fest verbunden ist, welches Ringlager (13) ein von dem anderen Schenkel (8, 34) umfasstes Drehlager für diesen Schenkel (8) bildet und dessen Drehebene (15) zur Drehebene (16) des Achsringes (14) durch Verdrehen des Einstellrings (12) um einen solchen Winkel verschwenkbar ist, dass der von dem Achsring (14) getragene Schenkel (7, 35) und mit ihm das von ihm gehaltene Glied (5, 31) je nach Verdrehwinkel des Einstellrings (12) gegen den mit dem Ringlager (13) verbundenen Schenkel (8, 34) eine Schwenklage dieses Gliedes (5, 31) gegenüber der gestreckten Normallage einnimmt.

2. Orthese nach Anspruch 1 zur Korrektur der Stellung der Großzehe (5) mit zwei Schenkeln (7, 8), die sich im angelegten Zustand vom Mittelfuß (28) zur Großzehe (5) an der Innenseite des Fußes (1) erstrecken und am Mittelfuß (28) und an der Großzehe (5) durch jeweils eine Manschette (3, 4) gehalten sind.

3. Orthese nach Anspruch 1 zur Korrektur der Stellung des Kniegelenks (32) mit zwei Schenkeln (34, 35), die sich im angelegten Zustand vom Oberschenkel (30) über das Kniegelenk (32) zum Unterschenkel (31) erstrecken, am Oberschenkel (30) und Unterschenkel (31) durch jeweils eine Manschette (36, 37) gehalten sind und durch ein dem Kniegelenk (32) benachbartes Drehgelenk (6) miteinander verbunden sind.

4. Orthese nach einem der Ansprüche 1 bis 3, **dadurch gekennzeichnet, dass** der den Achsring (14) umfassende Schenkel (7, 35) mit einer Ringskala (18) versehen ist, die den Verdrehwinkel des Ringlagers (13) und damit die Schwenklage des anderen Schenkels (8, 34) anzeigt.

5. Orthese nach einem der Ansprüche 1 bis 4, **dadurch gekennzeichnet, dass** die gelenkige Verbindung von den beiden Schenkeln (7, 8; 34, 35) und dem Einstellring (12) durch einen in den Achsring eingedrückten Spreizring (22) gesichert ist.

6. Orthese nach einem der Ansprüche 1 bis 5, **dadurch gekennzeichnet, dass** der den Achsring (14) umfassenden Schenkel (7, 35) mit einer wahlweise anbringbaren Verdrehsicherung (24) versehen ist.

7. Orthese nach einem der Ansprüche 1 bis 6, **dadurch gekennzeichnet, dass** die Schenkel (7, 8; 34, 35) auf ihrer dem Glied zugewandten Seite mit einer Polsterung (9, 10) versehen sind.

8. Orthese nach einem der Ansprüche 1 bis 7, **dadurch gekennzeichnet, dass** der Achsring (14) und das Ringlager (13) einstückig ausgebildet sind.

## Claims

1. Orthosis (2) for correcting the position of a body joint, said orthosis (2) extending over the two members (5, 28; 30, 31) connected by the body joint and being provided with two arms (7, 8; 34, 35) forming the orthosis (2), said arms (7, 8, 34, 35) each being held on the respective member (5, 28; 30, 31) by an attachment means (3, 4; 36, 37) and being interconnected by a pivot joint (6) adjacent to the body joint, **characterized in that**, for the bending required during normal movement, the pivot joint (6) is formed by an axle ring (14) embraced by the one arm (7, 35), said axle ring (14) being fixedly connected to a ring bearing (13) eccentric with respect to the axle ring (14) to form a central adjusting ring (12), said ring bearing (13) forming a pivot bearing for the other arm (8, 34), said pivot bearing being embraced by said other arm (8), wherein the rotation plane (15) of said ring bearing (13) with respect to the rotation plane (16) of the axle ring (14) is pivotable through rotation of the adjusting ring (12) by such an angle that the arm (7, 35) supported by the axle ring (14) - and, with it, the thereby held member (5, 31) - assume a pivot position of said member (5, 31) in relation to the extended normal position, depending on the rotation angle of the adjusting ring (12) with respect to the arm (8, 34) connected to the ring bearing (13).

2. Orthosis according to claim 1 for correcting the position of the big toe (5) with two arms (7, 8), said two arms (7, 8) extending in applied state from the metatarsus (28) to the big toe (5) on the inside of the foot (1) and being secured to the metatarsus (28) and to the big toe (5) by respective cuffs (3, 4).

3. Orthosis according to claim 1 for correcting the position of the knee joint (32) with two arms (34, 35), said two arms (34, 35) extending in applied state from the thigh (30) through the knee joint (32) to the lower leg (31), being secured to the thigh (30) and lower leg (31) by respective cuffs (36, 37) and being interconnected by a pivot joint (6) adjacent to the knee joint (32).

4. Orthosis according to any one of claims 1 to 3, **characterized in that** the arm (7, 35) embracing the axle ring (14) is provided with an annular scale (18), said annular scale (18) indicating the rotation angle of the ring bearing (13) and therefore the pivot position of the other arm (8, 34).

5. Orthosis according to any one of claims 1 to 4, **characterized in that** the articulated connection of the two arms (7, 8; 34, 35) and the adjusting ring (12) is secured by an expanding ring (22) pressed into the axle ring.

6. Orthosis according to any one of claims 1 to 5, **characterized in that** the arm (7, 35) embracing the axle ring (14) is provided with a selectively attachable anti-rotation means (24).

7. Orthosis according to any one of claims 1 to 6, **characterized in that** the arms (7, 8; 34, 35)) are provided on their side facing the member with respective pads (9, 10).

8. Orthosis according to any one of claims 1 to 7, **characterized in that** the axle ring (14) and the ring bearing (13) are integral with each other.

## Revendications

1. Orthèse (2) servant à corriger la position d'une articulation du corps, ladite orthèse s'étendant sur les deux membres (5, 28 ; 30, 31) reliés par l'articulation du corps et étant munie de deux plaques (7, 8 ; 34, 35) qui forment l'orthèse (2) et qui sont maintenues contre chacun des membres (5, 28 ; 30, 31) par un moyen de fixation (3, 4 ; 36, 37) et sont reliées entre elles par une articulation tournante (6) voisine de l'articulation du corps, **caractérisée en ce que** l'articulation tournante (6) assurant la flexion requise en déplacement normal est formée par une bague axiale (14) entourée par l'une des plaques (7, 35) et solidarisée à un palier annulaire (13) excentré par rapport à la bague axiale (14) pour former une bague centrale de réglage (12), ledit palier annulaire (13) formant un pivot destiné à l'autre plaque (8, 34) et entouré par celle-ci, et son plan de rotation (15) pouvant pivoter par rapport au plan de rotation (16) de la bague axiale (14) en tournant la bague de réglage (12) d'un angle tel que, en fonction de l'angle de rotation de la bague de réglage (12), la plaque (7, 35) portée par la bague axiale (14) et, avec elle, le membre (5, 31) maintenu par celle-ci adoptent, par rapport à la plaque (8, 34) reliée au palier annulaire (13), une position de pivotement de ce membre (5, 31) par rapport à la position normale tendue.

2. Orthèse selon la revendication 1 pour corriger la position du gros orteil (5), comprenant deux plaques (7, 8) qui, à l'état appliqué, s'étendent du métatarse (28) au gros orteil (5) contre la face interne du pied (1) et sont chacune maintenues contre le métatarse (28) et contre le gros orteil (5) par un manchon (3, 4).

3. Orthèse selon la revendication 1 pour corriger la position de l'articulation du genou (32), comprenant deux plaques (34, 35) qui, à l'état appliqué, s'étendent de la cuisse (30) à la jambe (31) en passant par l'articulation du genou (32), sont chacune maintenues contre 1a cuisse (30) et la jambe (31) par l'intermédiaire d'un manchon (36, 37) et sont reliées entre elles par l'intermédiaire d'une articulation tournante (6) voisine de l'articulation du genou (32).

4. Orthèse selon une des revendications 1 à 3, **caractérisée en ce que** la plaque (7, 35) entourant 1a bague axiale (14) est munie d'une graduation annulaire (18) qui indique l'angle de rotation du palier annulaire (13) et ainsi la position de pivotement de l'autre plaque (8, 34).

5. Orthèse selon une des revendications 1 à 4, **caractérisée en ce que** la liaison articulée des deux plaques (7, 8 ; 34, 35) et de la bague de réglage (12) est sécurisée par une bague d'écartement (22) enfoncée dans la bague axiale.

6. Orthèse selon une des revendications 1 à 5, **caractérisée en ce que** la plaque (7, 35) entourant la bague axiale (14) est munie d'un moyen de blocage en rotation (24) pouvant être mis en place de manière quelconque.

7. Orthèse selon une des revendications 1 à 6, **caractérisée en ce que**, sur leur côté tourné vers le membre, les plaques (7, 8 ; 34, 35) sont pourvues d'un rembourrage (9, 10).

8. Orthèse selon une des revendications 1 à 7, **caractérisée en ce que** la bague axiale (14) et le palier annulaire (13) sont réalisés d'un seul tenant.
